# EUROPEAN PATENT APPLICATION

(11) **EP 2 371 340 A1**
(43) Date of publication of application: **05.10.2011**
(21) Application number: 09830186.4
(22) Date of filing: 02.12.2009
(51) Int. Cl.: A61J 1/05, A24F 47/00, A61J 1/06, A61M 15/06, A61P 25/30

(54) **INSTRUMENT FOR ALLEVIATING ADDICTIVE DRUG CRAVING, METHOD FOR USING SAME AND METHOD FOR TREATING ADDICTIVE DRUG DEPENDENCE**

(30) Priority: 02.12.2008 JP 2008307447; 13.02.2009 JP 2009031262
(71) Applicant: Hirai, Shinji, Chiba-shi, Chiba 265-0066 (JP)
(72) Inventor: Hirai, Shinji, Chiba-shi, Chiba 265-0066 (JP)
(74) Representative: TBK
(86) International application number: PCT/JP2009/006523
(87) International publication number: WO 2010/064416

(57) **Abstract**

Provided is an instrument for alleviating addictive drug craving which can be safely administered to an addictive drug-dependent patient by a definite method in practice without causing any undesirable effects on therapy and terminate a series of behaviors through the administration so as to achieve an excellent effect on the therapy for alleviating addictive drug craving. An instrument for alleviating addictive drug craving (11) which comprises a pseudo injection agent (31) enclosed in a pseudo container (21). To the outer face of the pseudo container (21), which is an ampule-shaped container being similar in appearance and structure to a container of an addictive drug (X), a pseudo injection agent label (41), which is almost the same in constitution to a product label of the container of the addictive drug (X) except having the term "PSEUDO" added hereto, is attached. The pseudo injection agent (31) is similar in appearance to an injection agent of the addictive drug (X) but free from the active ingredient of the addictive drug (X).

## Description

### TECHNICAL FIELD

The present invention relates to an instrument for alleviating addictive drug craving, a method for using the same, and a method for treating addictive drug dependence.

### BACKGROUND ART

In recent years, the number of drug dependent patients has been continuing to increase steadily due to repetitive abuse of various addictive drugs as well as stimulants and a wide variety of treating methods have been tried to date on drug dependent patients. As one of such treating methods, the present inventor formerly proposed a treating method that is aimed at alleviating the activation of chaining of first signal type conditional reflexes by preparatorily giving a stimulant dependent patient a conditional stimulus for regenerating an action of consuming a stimulant and actually repeating the act of keeping him from consuming the stimulant thereby depriving the stimulant dependent patient of the significance of the conditional stimulus (see Non-Patent Document 1.)

Since the stimulant dependent patient repeats the process from obtaining a stimulant to actually consuming it, the face of the drug pusher recognized during the process, the pack containing the stimulant, the syringe used during the injection of the stimulant, etc. stimulate the independent circuits of first signal type conditional reflexes that govern the individual actions constituting a series of behaviors of searching and consuming the stimulant. For this reason, in the stimulant dependent patient, just a look at any of the face of the drug pusher, the pack containing the stimulant, and the syringe induces a conditional reflex in the first signal type and tends to commence an action governed by the conditional reflex. At this time, the friction between the promotion of a consuming behavior due to the operation of the first signal type conditional reflex and the alleviation of an consuming behavior due to the thought of the passive judgment of the situation allowing the stimulant to be neither procured nor consumed or the active plan of withdrawing the use of a stimulant (second signal type conditional reflex) is considered to be perceived by the stimulant dependent patient as an impulse toward a stimulant. The aforementioned treating method, therefore, is aimed at alleviating the craving of the stimulant dependent patient for a stimulant by giving the patient such a conditional stimulus as described above or inducing reflexes with a conditional stimulus and regenerating part of a series of actions and inducing extinction of the habit by repeating the act of keeping the patient from consuming the stimulant and precluding the activation of chaining of first signal type conditional reflexes even when a conditional stimulus is imparted.

The aforementioned treating method, however, has a problem that since a genuine stimulant desired to be used cannot be injected, the series of behaviors due to the activation of chaining of the first signal type conditional reflexes cannot be terminated and consequently the craving cannot be effectively alleviated. The inventors, therefore, have developed a pseudo syringe which is not provided with a hollow needle, allows the pseudo reflux of blood to be visually recognized without requiring the hollow needle to be plunged into the blood vessel, and enables the patient to acquire a conditional stimulus approximating the act of injection (see Patent Document 1.) The application of this pseudo syringe to the aforementioned treating method has enabled acquisition of a considerable degree of treating effect.

In the treating method using this pseudo syringe, however, because a genuine syringe having aspirated the solution of a stimulant in advance must be subsequently shifted to the pseudo syringe mentioned above, namely because the syringe having aspirated the stimulant solution cannot be used as is for the purpose of effecting injection, the problem arises that this fact constitutes a factor of inhibiting the series of actions of consuming the stimulant, disturbs the fluidity of consuming actions, and disables perfect regeneration of the consuming actions.

### PRIOR ART DOCUMENT

### PATENT DOCUMENT

Patent Document 1: Patent Application 2007-268142

### NON-PATENT DOCUMENT

Non-Patent Document 1: Shinji Hirai, "Therapy for alleviating conditional reflexes due to repetitive consumption of stimulant", Psychiatry, 2008, 13(1), p. 1 - 13

### DISCLOSURE OF THE INVENTION

### PROBLEM TO BE SOLVED BY THE INVENTION

Incidentally, there exist numerous addictive drugs such as narcotics and hallucinogens besides stimulants. The treating method mentioned above can be applied to the patients dependent on these addictive drugs. When the genuine addictive drug is adopted for use, however, this method confronts the problem that the drug cannot be actually administered to the drug dependent patient and the patient cannot expect any sufficient treating effect as in the case of the stimulant dependent patient.

The present invention has been produced in view of the problems confronted by the conventional techniques described above and is aimed at providing an instrument for alleviating addictive drug craving which can be safely administered to an addictive drug dependent patient by a definite method in practice without causing any undesirable effect on therapy and terminates a series of behaviors through the administration so as to achieve an excellent effect on the therapy for alleviating addictive drug craving.

### MEANS FOR SOLVING PROBLEM

The aforementioned object of this invention is accomplished by the following means.

(1) An instrument for alleviating addictive drug craving, possessing a visual appearance similar to the form of an addictive drug usually consumed by an addictive drug dependent patient and comprising a pseudo addictive drug not containing the effective component of said addictive drug.

(2) An instrument for alleviating addictive drug craving set forth in (1), wherein said addictive drug is antipsychotic agent, antianxiety agent, antimanic psychosis agent, stimulator for central nervous system, sleep inducing agent, calmative narcoleptive agent, antihistamic agent, overall cold agent, antitussive agent, anesthetic agent, lenitive agent, hallucinogenic agent, antihypnotic agent, tobacco, agent for aiding smoke prohibition, hormone, alcoholic beverage, organic solvent, or organic fuel.

(3) An instrument for alleviating addictive drug craving set forth in (1) or (2), wherein said pseudo addictive drug contains physiological salt solution, grape sugar solution, distilled water, grape sugar, cane sugar, milk sugar, starch, dextrin, or saccharose.

(4) An instrument for alleviating addictive drug craving set forth in any one of (1) - (3), wherein said pseudo addictive drug comprises a pseudo preparation possessing a visual appearance similar to the preparation of said addictive drug.

(5) An instrument for alleviating addictive drug craving set forth in (4), wherein said pseudo preparation is aerosol, internal liquid medicine, extract, elixir, capsule, granules, pills, ophthalmic ointment, suspension, emulsion, agent for percutaneous absorption, suppository, abstract, spirit, tablet, syrup, infusion, decoction, injection, patch, tincture, ophthalmic solution, troche, ointment, poultice, aromatic water, liniment, limonade, fluidextract, lotion, catapasm, paste, collutorium, malagma, liniment, abluent, clyster, bath liquid, tea, or lincture.

(6) An instrument for alleviating addictive drug craving set forth in (4) or (5), wherein said pseudo addictive drug possesses a display indicating this drug to be a counterfeit attached to the surface of said pseudo preparation.

(7) An instrument for alleviating addictive drug craving set forth in any one of (1) - (6), wherein said pseudo addictive drug is enclosed with a pseudo container similar in visual appearance and structure to the container of said addictive drug.

(8) An instrument for alleviating addictive drug craving set forth in (7), wherein said pseudo container is ampoule, vial, bottle, bag, syringe, sheet, stick, tube, container, pouch, or charta.

(9) An instrument for alleviating addictive drug craving set forth in (7) or (8), wherein said pseudo container is made of glass, rigid plastic, flexible plastic, metal, paper, or a combination thereof.

(10) An instrument for alleviating addictive drug craving set forth in any one of (7) - (9), wherein said pseudo addictive drug possesses a display indicating this drug to be a counterfeit attached to the visible surface of said pseudo container.

(11) An instrument for alleviating addictive drug craving set forth in any one of (7) - (10), wherein a pseudo package similar in visible appearance and structure to the package applied to said container is applied to said pseudo package.

(12) An instrument for alleviating addictive drug craving set forth in (11), wherein said pseudo package is a pouch, a box, or a sheet which is made of paper or plastic.

(13) An instrument for alleviating addictive drug craving set forth in (11) or (12), wherein a display indicating said pseudo addictive drug to be a counterfeit is applied to the visible surface of said pseudo package.

(14) A method for using an instrument for alleviating addictive drug craving which possesses a visible appearance similar in form to said addictive drug usually consumed by an addictive drug dependent patient and contains none of the active component of said addictive drug, which method comprises the first step of procedure for causing said pseudo addictive drug to be consumed by a prescribed method.

(15) A method for using an instrument for alleviating addictive drug craving set forth in (14), which further comprises the second step of procedure for opening said pseudo container when said pseudo addictive drug is enclosed in a pseudo container similar in visible appearance and structure to the container of said addictive drug.

(16) A method for using an instrument for alleviating addictive drug craving set forth in (15), which further comprises the third step of procedure for undoing said pseudo package when said pseudo container has applied thereto a pseudo package similar in visible appearance and structure to the package of said container.

(17) A method for using an instrument for alleviating addictive drug craving set forth in (15) or (16), wherein said first step of procedure further comprises the fourth step of procedure for inserting the hollow needle of a syringe into the pseudo container opened by said second step of procedure and aspirating said pseudo addictive drug in said container with said syringe when said pseudo addictive drug is an injection.

(18) A method for using an instrument for alleviating addictive drug craving set forth in (17), wherein said first step of procedure further comprises the fifth step of procedure for plunging into the patient's own body said hollow needle of said syringe which has aspirated said pseudo addictive drug by said fourth step of procedure and injecting said pseudo addictive drug.

(19) A method for using an instrument for alleviating addictive drug craving set forth in any one of (14) - (18), which has part or the whole of said first - fifth steps of procedure repetitively performed for a definite period of time or steadily.

(20) A method for curing dependence on an addictive drug by using an instrument for alleviation of addictive drug craving which possesses a visible appearance similar in form to said addictive drug usually consumed by an addictive drug dependent patient and contains none of the active component of said addictive drug, which comprises the first step of procedure for causing said dependent patient to consume said pseudo addictive drug by himself by a prescribed method.

(21) A method for curing dependence on an addictive drug set forth in (20), which further comprises the second step of procedure for causing said addictive dependent patient to open said pseudo container when said addictive drug is enclosed with said pseudo container similar in visible appearance and structure to the container of said addictive drug.

(22) A method for curing dependence on an addictive drug set forth in (21), which further comprises the third step of procedure for causing the addictive drug dependent patient to undo said pseudo package when said pseudo package has applied thereto a pseudo package similar in visible appearance and structure to the package of said container.

(23) A method for curing dependence on an addictive drug set forth in (21) or (22), wherein said first step of procedure further comprises the fourth step of procedure for causing said addictive drug dependent patient to plunge the hollow needle of the syringe into the pseudo container opened by said second step of procedure and aspirate said pseudo addictive drug with said syringe when said pseudo addictive drug is an injection.

(24) A method for curing dependence on an addictive drug set forth in (23), wherein said first step of procedure further comprises the fifth step of procedure for causing said addictive drug dependent patient to plunge into his own body said hollow needle of said syringe which has aspirated said pseudo addictive drug by said fourth step of procedure and inject said pseudo addictive drug.

(25) A method for curing dependence on an addictive drug set forth in any one of (20) - (24), which has part or the whole of said first - fifth steps of procedure repetitively performed by said addictive drug dependent patient for a definite period of time or steadily.

### EFFECT OF THE INVENTION

The instrument of the present invention for alleviating addictive drug craving uses a pseudo container which is similar in visible appearance and structure to the aforementioned container for addictive drug. When an addictive drug dependent patient views, grabs, or opens the pseudo container of the instrument of the present invention for alleviating addictive drug craving, he is enabled to acquire a conditional stimulus not different at all from what he acquires when viewing, grabbing, or opening the container of the pseudo addictive drug and obtain an excellent effect for the therapy of alleviating the addictive drug craving.

The instrument of the present invention for alleviating addictive drug craving has the aforementioned pseudo container enclose therein a pseudo preparation similar in visible appearance to the preparation of the aforementioned addictive drug. When an addictive drug dependent patient plunges the hollow needle into the opened pseudo injection container of the instrument of the present invention for alleviating addictive drug craving and aspirates the pseudo injection in the pseudo injection container with the syringe, therefore, he is enabled to acquire a conditional stimulus not different at all from what is obtained when aspirating the genuine injection of addictive drug with the syringe, when opening the pseudo container of tablets, taking out one of the tablets, and placing it into the mouth, when dissolving a pseudo granular preparation in a definite solvent and drinking the solution, when aspiring the solution with a definite appliance, or when opening the pseudo container of suppository, retrieving the pseudo suppository preparation, and inserting this preparation into the anus or vagina and obtain an excellent effect for the therapy for alleviating the addictive drug craving.

Further, the instrument of the present invention for alleviating addictive drug craving comprises enclosing therein a pseudo preparation not containing the effective component of the aforementioned preparation. When the pseudo preparation of the instrument of the present invention for alleviating addictive drug craving is consumed, it remains safe and does not exert any adverse effect on the therapy. By actually administering this pseudo preparation to an addictive drug dependent patient by a definite method, it is made possible to terminate a series of behaviors due to the chained actions of the first signal type conditional reflexes and obtain an excellent effect on the therapy for alleviating the addictive drug craving.

Further, since the instrument of the present invention for alleviating addictive drug craving comprises enclosing therein a pseudo preparation not containing the effective component of the aforementioned preparation, it can be manufactured less expensively than the genuine addictive drug and enabled to effect the therapy for alleviating the addictive drug craving at a lower cost than when the genuine addictive drug is used.

Since the instrument of the present invention for alleviating addictive drug craving comprises having the aforementioned pseudo container affixed thereto a pseudo package similar in visible appearance and structure to the package affixed to the aforementioned container, the addictive drug dependent patient is enabled by undoing the pseudo package affixed to the pseudo container of the instrument of the present invention for alleviating addictive drug craving to acquire a conditional stimulus not different at all from what is acquired when the package affixed to the container of the genuine addictive drug is undone and obtain an excellent therapeutic effect for alleviating the addictive drug carving.

Since the instrument of the present invention for alleviating addictive drug craving comprises having attached to the surface of the aforementioned addictive drug or to the external surface of the aforementioned container or the aforementioned pseudo package a display indicating that the aforementioned preparation is a counterfeit, the addictive drug dependent person who happens to be a person engaging in medical care is disabled to mistake the instrument of the present invention for alleviating addictive drug craving for the genuine addictive drug and enabled to prevent therapeutic malpractice.

### BRIEF DESCRIPTION OF THE DRAWINGS

[Fig. 1] This is a schematic cross section illustrating the general structure of an instrument 11 for alleviating addictive drug craving according to the first form of carrying out the present invention.
[Fig. 2] This is a schematic front view illustrating the visual structure of a pseudo container 21.
[Fig. 3] This is a schematic front view illustrating the structure of a pseudo injection agent label 41.
[Fig. 4] This is a schematic oblique view illustrating the structure of a pseudo package 51.
[Fig. 5] This is a view illustrating the visible structure of an instrument 12 for alleviating addictive drug craving 12 according t the second mode for carrying out the present invention; (A) a schematic front view and (B) a schematic rear view.
[Fig. 6] This is a schematic front view illustrating the visible structure of a pseudo tablet 32
[Fig. 7] This is a schematic front view illustrating another example of the pseudo container.
[Fig. 8] This is a schematic front view illustrating another example of the pseudo injection agent label.
[Fig. 9] This is a schematic front view illustrating another example of the injection agent label.

### MODE(S) FOR CARRYING OUT THE INVENTION

The modes for carrying out the present invention will be described in detail herein below with reference to the drawings.

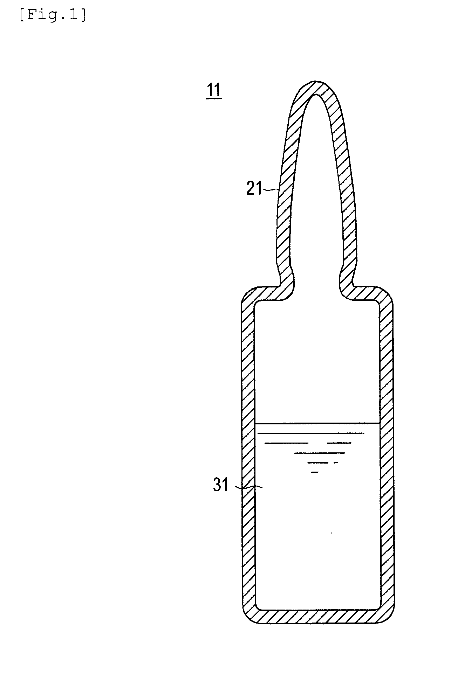

Fig. 1 is a schematic cross section illustrating the general structure of an instrument 11 for alleviating addictive drug craving according to the first mode of carrying out the present invention. As illustrated in Fig. 1, the instrument 11 for alleviating addictive drug craving according to the present mode for carrying out the present invention comprises having a pseudo container 21 enclose a pseudo injection agent 31 therein.

The pseudo container 21 is an ampoule-shaped container similar in visible appearance and structure to the container for an addictive drug (addictive drug X). That is, the pseudo container 21 bears a similarity in shape, color, pattern, dimension, weight, etc. to the (ampoule-shaped) injection container for the addictive drug X and possesses a similar structure. Consequently, an addictive drug dependent patient is enabled by viewing, grabbing, or opening the pseudo container 21 of the instrument for alleviating addictive drug craving 11 to acquire a conditional stimulus nearly identical to the conditional stimulus that is obtained when the injection container of the genuine addictive drug X is viewed, grabbed, or opened. The raw material for the pseudo container 21 is not especially limited but may be selected from among glass, rigid plastics, and flexible plastics, for example. For the same reason, it is preferable to use a similar raw material for the injection container of the addictive drug X.

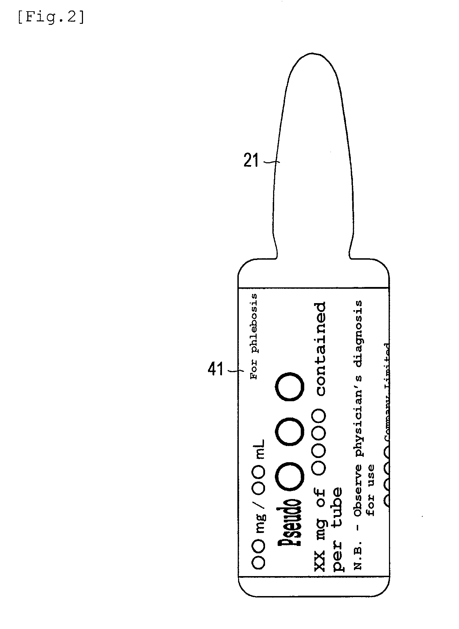

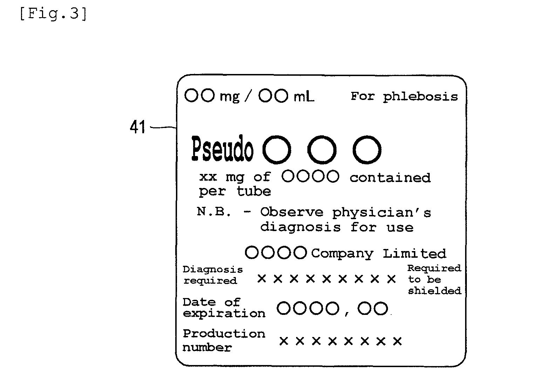

Fig. 2 is a schematic front view illustrating the visible structure of the pseudo container 21. As illustrated in Fig. 2, the pseudo container 21 has a pseudo injection agent label 41 attached to the visible surface of pseudo container 21. As illustrated in Fig. 3, this pseudo injection agent label 41 has a visible appearance resembling a product label attached to the injection container of an addictive drug X and has a structure almost identical with the product label attached to the injection container of the addictive drug X except the letter "pseudo" is prefixed to the product name "o o o " of the addictive drug X. This letter "pseudo" indicates that the pseudo injection agent 31 enclosed in the pseudo container 21 of the instrument 11 for alleviating addictive drug craving is not a genuine injection agent of addictive drug X but a pseudo injection agent. Even when the addictive drug dependent patient happens to be a person engaging in medical care, therefore, he is no longer suffered to mistake the instrument 11 for alleviating addictive drug craving for a genuine addictive drug X. Thus, such a medical care accident that occurs when an addictive drug dependent patient mistakes a genuine addictive drum X for the instrument 11 for alleviating addictive drug craving and puts it to his own use or conversely when the patient performing a medical care mistakes the instrument 11 for alleviating addictive drug craving for a genuine addictive drug X and uses it on a patient can be prevented. Since the addictive drug dependent patient knows from the beginning the instrument 11 for alleviating addictive drug craving as a counterfeit and uses it in the therapy for alleviating addictive drug craving, the fact that the pseudo container 21 has a display indicating it to be a counterfeit does not influence the treating effect.

The pseudo injection agent 31 is in a liquid state and has a visible appearance similar additionally in color, viscosity, etc. to the injection agent of an addictive drug X. As a result, the addictive drug dependent patient is enabled by inserting the hollow needle of a syringe into the opened pseudo container 21 of the instrument 11 for alleviating addictive drug craving and aspirating the pseudo injection agent 31 with the syringe to acquire a conditional stimulus nearly identical to the conditional stimulus which is obtained when the injection agent of a genuine addictive drug X is aspirated with the syringe.

The pseudo injection agent 31 does not contain an effective component of the injection agent of the addictive drug X and comprises physiologically acceptable components harmless to human body. Consequently, since the ingestion of the pseudo injection agent 31 is safe and exerts no adverse influence on the therapy and since the pseudo injection agent 31 is therefore enabled to be actually injected into an addictive drug dependent patient and the series of actions due to the chained behaviors of the first signal type conditional reflexes can be terminated, the therapy for alleviating the addictive drug craving can acquire an excellent effect. As examples of the pseudo injection agent 31 of this quality, a substance comprising components similar to the components other than the effective component of the addictive drug X, such alternative solutions of the effective component of the addictive drug X as physiological salt solution and grape sugar, and mixtures of distilled water with additive agents may be cited.

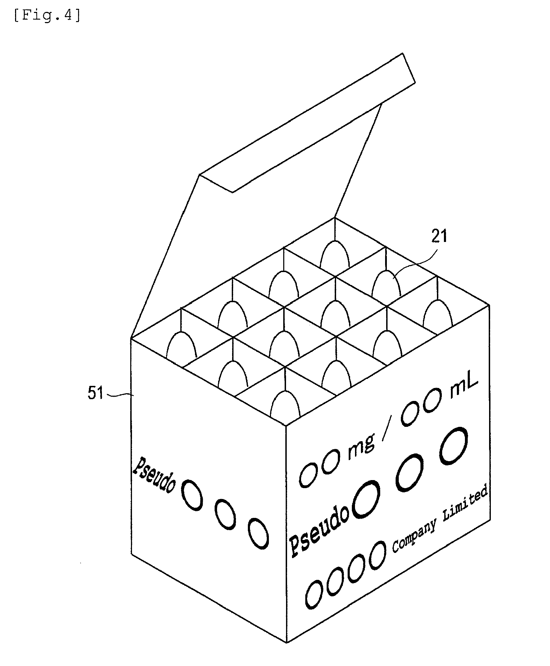

Incidentally, when the injection container of the addictive drug X is furnished with a package, the pseudo container 21 may be furnished with a pseudo package similar in visible appearance and structure to the package mentioned above. As a result, the addictive drug dependent patient is enabled by opening the package attached to the pseudo container 21 to acquire a conditional reflex nearly similar to the conditional reflex obtained when the package attached to the injection container of the genuine addictive drug X is opened. As examples of the pseudo package of this quality, boxes, pouches, tying bands (capable of tying the whole or part of the pseudo container 21), and the like which are formed of paper, plastic, etc. may be cited. This pseudo package may be an assembly package holding a plurality of pseudo injection containers as illustrated in Fig. 4 or an independent package holding one injection container each (not illustrated). Incidentally, the pseudo package may have attached thereto a display indicating that the pseudo injection agent 31 enclosed therein is a counterfeit as illustrated in Fig. 4 as in the case of the pseudo injection agent label 41.

Next, the method for using the instrument 11 for alleviating addictive drug craving and the method for treating the dependence on the addictive drug by the use of the instrument 11 for alleviating addictive drug craving will be explained. First, the patient who is dependent on addictive drug is given the instrument 11 for alleviating addictive drug craving, made personally to grab in his own hand the instrument 11 for alleviating addictive drug craving, open the pseudo package thereof, and remove the pseudo package 21 (Step 1). Then, the patient is made to open the pseudo container 21 by inflicting a score in the joint part between the barrel part and the head part of the removed pseudo container 21 with a glass cutter, for example, and breaking off the head part (Step 2). Next, the patient is made to insert the hollow needle of the syringe into the opened pseudo container 21 and aspirate the pseudo injection agent 31 in the pseudo container 21 with the syringe (Step 3). And, the patient is made to plunge the hollow needle of the syringe into the vein of his own arm and inject the pseudo injection agent 31 and, after completion of this injection, extract the syringe needle from his body (Step 4). Finally, the patient is made to clear away all the appliances used in Steps 1 - 4.

On the part of the person who consumes an addictive drug repetitively, originally the individual actions constituting a series of behaviors of obtaining and consuming an addictive drug are curbed by thinking and rewarded after the series of behaviors end. When these experiences are repeated, a certain series of behaviors are conditioned and the individual actions constituting the series of behaviors come to be controlled by a circuit corresponding to the first signal type conditional reflex. The individual circuits of the conditional reflex assume a state tending to constitute a chain with a mechanism causing one circuit to stimulate the next circuit, cause the chain of conditional reflexes to undergo an active reaction in the presence of a stimulus associated with an addictive drug, and allow the series of behaviors of consuming an addictive drug to advance. On the part of a person who has repetitively consumed a substance destined to award a pharmacologic action, the conditional reflex that is given to him because he performs actions constituting a series of behaviors (such as, for example, the actions of grabbing an addictive drug in his hand or opening the container thereof), or passively hears a spoken language "addictive agent" and views a syringe is considered to actuate the circuit of the first signal type conditional reflex created in the cerebrum and tend to promote the series of behaviors of consuming an addictive drug. Thus, a person who tries to conduct therapy for alleviating drug dependence repeats the intentional replication of behaviors of obtaining and consuming an addictive drug by the use of the instrument 11 for alleviating addictive drug craving because he is unable to acquire the reward which arises when he consumes a genuine addictive drug. In the initial stage of the act of therapy, therefore, this act causes the chain of the first signal type conditional reflexes to be actuated automatically and enables advance of behaviors similar to obtaining and consuming a substance. Since no reward is given after each series of behaviors in the act of therapy, the conditioning of the therapy is repressed, namely the practicability of the chain of the first signal type conditional reflexes gradually becomes repressed. Finally, the craving for an addictive drug due to the conditional reflexes ends up in extinction.

First in the therapy for alleviating addictive drug craving, it is preferable to have Steps 1 - 4 repeated intensively in such an environment as a hospital isolated from the society open for craving for an addictive drug at a frequency of 5 to 15 times daily up to a total of at least 300 to 500 times. In the initial stage of the therapy, however, Steps 1 - 4 are preferred to be wholly repeated because the series of behaviors of searching, obtaining, and consuming a drug are performed from the start of the behaviors to the behavior immediately before obtaining the reward of the drug. In the intermediate stage∼ later stage of the therapy wherein the driving property of the conditional reflex has been reduced, the quality and quantity of stimulus may be regulated by discontinuing the act carried out halfway in Steps 1 - 4 or carrying out only part of the act. Steps 1-4, besides being repeated during a definite period of incentive therapy, are preferably repeated steadily at a stated timing after the period mentioned above for the purpose of preventing the addictive drug craving and preventing recurrence of the craving.

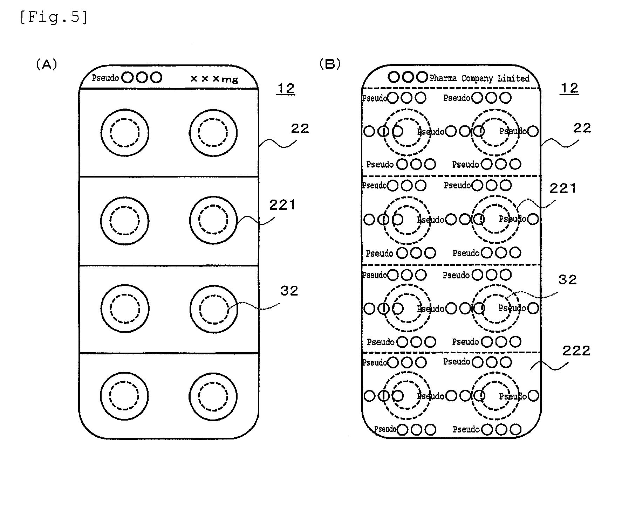

Fig. 5 is a drawing illustrating the visual configuration of an instrument 12 for alleviating addictive drug craving according to the second mode of carrying out the present invention; (A) a schematic front view and (b) a schematic rear view. The instrument 12 for alleviating addictive drug craving, as illustrated in Fig. 5 (A) and (B), comprises having pseudo tablets 32 occluded in a pseudo container 22.

The pseudo container 22 is a sheet-shaped preparation container similar in external appearance and structure to a container for addictive drug Y tablets. Specifically, it has the pseudo tablets 32 contained in a plurality of convexes formed in a plastic sheet 221 and has the pseudo tablets 32 occluded in the convexes by having an aluminum foil 222 superposed thereon. Specifically, the pseudo container 22 is similar in shape, color, pattern, dimension, weight, etc. to the container (sheet-shaped) for addictive drug Y tablets and possesses a structure similar thereto. Similarly to the instrument 11 for alleviating addictive drug craving, therefore, the instrument 12 for alleviating addictive drug craving is capable of acquiring a conditional reflex nearly equal to the conditional reflex which is obtained by the container for addictive drug Y tablets. The raw material for the pseudo container 22 does not need to be especially limited but allows use of flexible plastic, metal, paper, combinations thereof, etc. for example. For the same reason, it is preferable to use a raw material similar to the raw material for the container of addictive drug Y tablets.

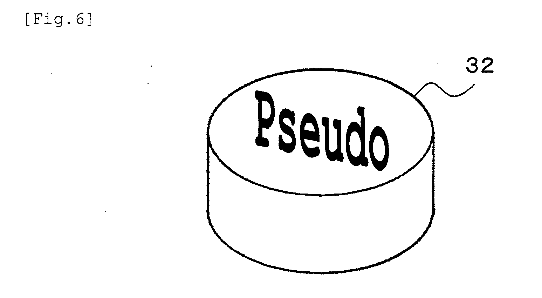

Fig. 6 is a schematic front view illustrating the visual configuration of the pseudo tablet 32. As illustrated in Fig. 6, the pseudo container 22 has the surface and the rear surface thereof each furnished with a configuration nearly equal to what is printed on the container for addictive drug Y tablets except the letter "pseudo" is prefixed to the product name "o o o " printed on the container for addictive drug X tablets. This letter "pseudo" manifests an effect similar to the effect of the instrument 11 for alleviating addictive drug craving.

The pseudo agent 32 is solid and possesses a visible appearance similar inclusively in color, hardness, etc. to the tablet of an addictive drug Y except the pseudo agent 32 has the letter "pseudo" imprinted on the top surface. As a result, the instrument 12 for alleviating addictive drug craving is capable of acquiring a conditional reflex nearly identical to the conditional reflex obtained by the tablet of addictive drug X, similarly to the instrument 11 for alleviating addictive drug craving. Incidentally, the pseudo tablet 32 does not need to have the letter "pseudo" imprinted on the top surface thereof.

Further, the pseudo tablet 3 avoids containing the effective component of an addictive drug Y similarly to the instrument 11 for alleviating addictive drug craving and manifests an effect similar to the effect of the instrument 11 for alleviating addictive drug craving. As examples of the raw material for the pseudo tablet 32 of this quality, the substances composed of components similar to the components other than the effective component of an addictive drug Y, alternate substance for the effective component of an addictive drug Y such as grape sugar, cane sugar, etc., and additives for such excipients as milk sugar, starch, dextrin, saccharose, etc. may be cited.

When this container for addictive drug Y tablets is furnished with a package, the instrument 12 for alleviating addictive drug craving may be furnished with a pseudo package similarly to the instrument 11 for alleviating addictive drug craving. As a result, it is made capable of acquiring a conditional reflex nearly identical with the conditional reflex which is obtained with a pseudo package, similarly to the instrument 11 for alleviating addictive drug craving.

Next, the method for using the instrument 12 for alleviating addictive drug craving and the method for treating the dependence on an addictive drug by the use of the instrument 12 for alleviating addictive drug craving will be explained. First, the patient who is dependent on addictive drug is given the instrument 12 for alleviating addictive drug craving, made personally to grab in his own hand the instrument 12 for alleviating addictive drug craving, open the pseudo package thereof, and remove the pseudo package 22 (Step 5). Then, the patient is made to open the pseudo container 22 by strongly depressing with the tip of his finger the convex of the plastic sheet 221 of the removed pseudo container 22 enclosing the pseudo tablet 32 and breaking the aluminum foil 222 on the rear surface (Step 6). Next, the patient is made to remove the pseudo tablet 32 from the convex of the pseudo container 22 (Step 7). And, the patient is made to place the pseudo tablet 32 into his own mouth (Step 8). Finally, the patient is made to clear away all the appliances used in Steps 5 - 8.

The therapy using the instrument 12 for alleviating addictive drug craving is carried out in the same way as the instrument 11 for alleviating addictive drug craving.

As examples of the addictive drug X and the addictive drug Y which are objects of imitation by the instrument of the present invention for alleviating addictive drug craving, the antipsychotic agents comprising the typical antipsychotic agents including the perphenazinephenothiazine system such as chlorpromazine, levomepromazine, thioridazine, fluphenazine, propericyaszine, etc., the butyrophenone system such as haloperidol, bromperidom, timiperone, spiperone, pimozide, etc., the benzamide system such as sulpiride, sultopride, nemonapride, etc., the indole system such as oxypertine, etc., and zotepine, mosapramine, clocapramine, etc., and the antipsychotic agents including the serotonin dopamine antagonistic (SDA) agents such as risperidone, perospirone, blonanserin, ziprasidone, sertindole, olanzapine, quetiapine, etc., the polyphyletic acceptor target antipsychotic agents (MARTA) such as the dibenzotiapine type such as quentiapine, and olanzapine, and the antipsychotic agents including the dopamine type stabilizing agents (DSS) such as aripiprazole, etc., the antianxiety agents comprising the benzodiazepin system such asetizolam, clotiazepam, flutazolam, lorazepam, arprazolam, bromazepam, oxazepam, fludiazeram, mexazolam, diazepam, cloxazolam, chlordiazepoxide, madazepam, oxazolam, flutoprazepam, ethyl loflazepate, and prazepam, etc., and the non-benzodiazepin system such as hydroxyzine, meprobamate, tandosspirone, etc., the antimanic-depressive psychosis agents such as lithium carbonate, sodium valproate, carbanazeoube, and sultopride hydrochloride, the stimulators for central nervous system such as methamphetamine hydrochloride (phenylmethylaminopropane), amphetamine, dextro amphetamine (d-amphetamine), pypradrol hydrochloride, methylphenidate hydrochloride, dexamethylphenidate, pemoline, adrafinil, and modafinil, etc., the sleep inducing agents comprising the ultrashort-time operating type calmative narcoleptive agents such as triazolam, zopiclone, and zolpidem tartrate, etc., the short-medial time operating type calmative narcoleptive agents such as etizolam, brotizolam, lormetazepam, and bromvalerylurea, etc., the medial time operating type calmative narcoleptive agents such as flunitrazepam, nitrazepam, nimetazepam, estazolam, quazepam, amobarbital, and aqueous chloral, etc., the long time operating type calmative narcoleptive agents such as flurazepam, phenobarbital, and haloxazolam, etc., and Vegetamin A/B, ramelteon, etc., the calmative narcoleptive agents such as barbital, benzoazepin, etc., the antiallergic minor tranquilizers comprising the diphenhydramine system such as diphenhydramine hydrochloride, diphenhydramine tannate, etc., the diphenylpyraline system such as diphenylpyraline teoclate, etc., the tripelennamine system such as clemastine fumarate, triprolidine hydrochloride, etc., the phenothiazine system such as chlorpheniramine dl-maleate, chlorpheniramine d-maleate, promethazine hydrochloride, alimemazine tartrate, etc., and hydroxyzine hydrochloride, hydroxyzine pamoate,etc., the antihistamic agents (inclusive of the overall common cold agents and the antitussive agents) comprising the pyrelysine system such as homochlorocyclizine hydrochloride, ciproheptazine hydrochloride, etc., and mequitazine, ketotifen fumarate, azelastine hydrochloride, oxatomide, emedastine fumarate, epinastine hydrochloride, ebastine, cetirizine hydrochloride, bepotastine besylate, fexofenadine hydrochloride, olopatadine hydrochloride, loratazine, etc., the ester-formanes the tic agents comprising cocaine, procaine, chloroprocaine, tetracaine, etc., the amide-form anesthetic agents comprising lidocaine, mepivacaine, dibucaine, bupivacaine, etc., the lenitive agents comprising morphine, calonal, isopropyl antipyrine, ibuprofen, aspirin, ethenzamide, rokisonine, voltaren, etc., the hallucinogenic agents comprising the chemical drugs such as lysergic acid dimethylamide (LSD), phencyclidine (PCP), etc., the natural drugs such as syrosibin, mescaline, harmaline, dimethyltryptamine (DMT), salvinoline A, ibotenic acid, muscimol, dimethyltryptamine (DMT), lysergic acid amide (LSA), ibogaine, etc., and the designers' drugs such as methylenedioxy methanefetamine (MDMA), MDA, 2C-B, 2C-T-2, 2C-T-4, 2C-T-7, 2C-I, α -methyl tryptamine (AMT), methylone, 5-MeO-DMT, 2,5-dimethoxy-4-ethylamphetamine (TMA), TMA-2, etc., the antihypnotic agents comprising phenylaminopropane (amphetamine), phenylmethylaminopropane (methanephetamine), and salts thereof, the tobaccos, the agents for aiding smoke prohibition comprising nicotine patch, nicotine tablet, nicotine gum, etc. , the agents for curing impotence comprising anastrozole, exemestane, estramustine, ethinyl estradiol, chlormadinone, goserelin, tamoxifen, dexamethasone, toremifene, bicalutamide, flutamide, prednizolone, fosfestrol, mitotane, methyltestosterone, medroxyprogesterone, mepitiostane, ryupurorerin, retrosole, sildenafilcitrate, valdenafil, tadarafiru, etc., the hormone agents comprising supplements such as proteins used for diet, cosmic treatment, carnal improvement, sexual function improvement, etc., the alcoholic beverages comprising Japanese sake, *shochu* (Japanese distilled liquor made from wheat, sweet potato, etc.), beer, wine, vodka, whisky, liqueur, etc., the organic solvents comprising thinner, toluene, adhesive, etc., and the organic fuels comprising cassette stove gas, lighter gas, gas packing, gasoline, lighter oil, etc. may be cited.

The instrument of the present invention for alleviating addictive drug craving is not restricted to the foregoing forms of embodiment but naturally allows addition of various alterations within the range that prohibits deviation of the essence of the present invention.

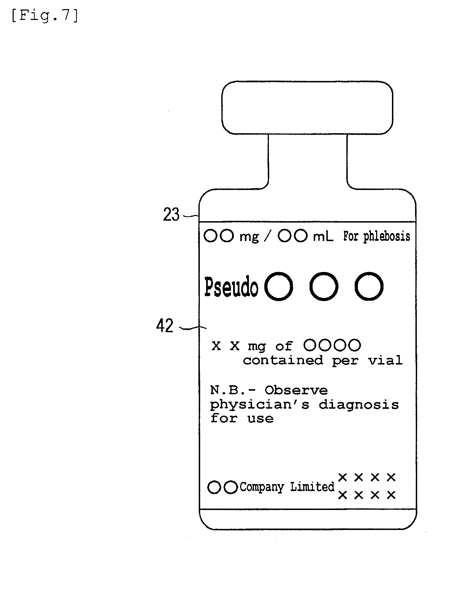

In the foregoing first form of embodiment, for example, the instrument of the present invention for alleviating addictive drug craving was explained by citing an ampoule-shaped pseudo injection container. The configuration of the pseudo injection container, however, is not limited to that of the cited example but may be suitably selected in conformity with the configuration of an injection container for the therapy of addiction to be simulated. As examples of the configuration of the pseudo injection container in the instrument of the present invention for alleviation of addictive drug craving, the shape of a vial illustrated in Fig. 7, the shape of a bottle, the shape of a bag, and the shape of a syringe may be cited besides the shape of an ampoule. As examples of the configuration of the pseudo tablet container, the shape of a stick, the shape of a tube, the shape of a container, the shape of a pouch, and the shape of a charta may be cited besides the shape of a sheet.

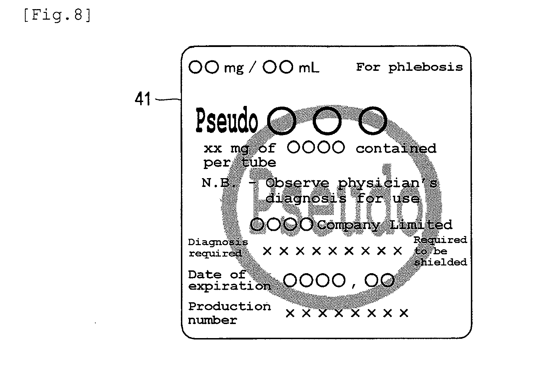

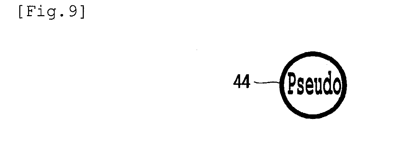

The letter "pseudo" on the pseudo injection agent label is not necessarily required to prefix the name of the product. It may be put in an optional blank portion of the label or may be displayed on another letter of the label in the form of a watermark letter as illustrated in Fig. 8. Further, the letter "pseudo" is not necessarily required to be printed on the pseudo injection agent label. As illustrated in Fig. 9, the letter "pseudo" may be formed in the shape of a label different from the pseudo injection agent label, in the shape of a seal, in the shape of a stamp, etc. and may be displayed by being mounted on the pseudo injection agent label or applied to an optional part other than the pseudo injection agent label of the pseudo injection container.

Further, in the instrument of the present invention for alleviating addictive drug craving, the display that indicates the pseudo injection agent to be a counterfeit is not required to be restricted on the condition that the user (the dependent patient) is enabled to distinguish the instrument of the present invention for alleviating addictive drug craving from a genuine addictive drug. Besides the letter "pseudo ( 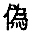 )" mentioned above, such letters as " 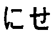 ", " 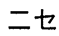 ", " 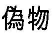 ", "FAKE", etc., such signs as "o ", "● ", "ⓞ ", "□", "■ ", "◇ ", "◆ ", "△ ", "▲ ", "∇ ", "▼ ", "☆ ", "★ ", " X ", " 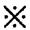 "_{,} "*". "?", "!", etc., and specific patterns may be used for the purpose of the display. Otherwise, the distinction may be attained by causing the color of the letter of the pseudo injection agent label or the color of the mount to differ from the color of the product label of a genuine addictive drug.

In the first form of embodiment described above, the case of having a product label applied to the injection container of a genuine addictive drug was explained by citing a pseudo injection container used therein. When no product label is applied to the injection container of a genuine addictive drug and a product name is directly printed on the injection container, for example, it goes without saying that the instrument for alleviating addictive drug craving according to the present invention is similarly configured.

Further, in the first form of embodiment described above, the method for using the instrument for alleviating addictive drug craving according to the present invention was explained by citing injection into the vein as an example. This method may be implemented by using other form of injection such as, for example, instillation. Instead of actually injecting the pseudo injection agent according to the present invention with a syringe, the act of pseudo injection may be carried out by using the instrument for alleviating addictive drug craving described in Patent Document 1, for example.

In the first and second forms of embodiment described above, the forms of therapeutic agents for use in the instrument of the present invention for alleviating addictive drug craving were explained by citing an injection agent and tablets as examples. The therapeutic agents may be those in other forms such as, for example, aerosol, internal liquid medicine, extract, elixir, capsule, granules, pills, ophthalmic ointment, suspension, emulsion, agent for percutaneous absorption, suppository, abstract, spirit, syrup, infusion, decoction, patch, tincture, ophthalmic solution, troche, ointment, poultice, aromatic water, liniment, limonade, fluidextract, lotion, catapasm, paste, collutorium, malagma, liniment, abluent, clyster, bath liquid, tea,lincture, etc. The pseudo addictive drug which the instrument of the present invention for alleviating addictive drug craving concerns is not necessarily required to be pharmaceutically prepared. It may be a product resulting from imitating an addictive drug which is not pharmaceutically prepared.

### INDUSTRIAL APPLICABILITY

As described above, the fact that a person repetitively consuming an addictive drug intentionally performs, by using the instrument of the present invention for alleviating addictive drug craving, an act of acquiring it, an act of undoing the pseudo package, an act of opening the pseudo container, an act of withdrawing the pseudo drug from the opened pseudo container by a prescribed method, an act of consuming the withdrawn pseudo drug by a prescribed method, etc. implies performance of a series of behaviors not different at all from the case of acquiring and consuming a genuine addictive drug. By this process, it is made possible to enable completion of the chain of operations up to the final reflecting circuit while ensuring inhibition of reward in the series of the first signal type conditional stimulus that validates the series of behaviors of acquiring and consuming a genuine addictive drug and enable safe performance of the process of inhibiting the first signal type conditional reflection that concerns validation of the series of behaviors. When an addictive drug dependent patient repeats the aforementioned acts for a definite period of time or steadily by the use of the instrument of the present invention for alleviating addictive drug craving, therefore, this instrument
manifests an unusually excellent effect in the therapy of alleviating the symptom of craving antipsychotic agents, antianxiety agents, antimanic-depressive psychosis agents, stimulants for central nervous system, sleep inducing agents, calmative narcoleptive agents, antihistamic agents (inclusive of the overall cold agents and the antitussive agents), anesthetic agents, lenitive agents, hallucinogenic agents, antihypnotic agents, tobaccos, agents for aiding smoke prohibition, hormones, alcoholic beverages, organic solvents, organic fuels, etc., in preventing the symptom of craving, and in preventing the recurrence of the symptom.

### DEFINITION OF SIGNS

- 11, 12: Instrument for alleviating addictive agent craving
- 21, 22, 23: Pseudo container
- 221: Plastic sheet
- 222: Aluminum foil
- 31: Pseudo injection agent
- 32: Pseudo tablet
- 41, 42, 43, 44: Pseudo injection agent label
- 51: Pseudo package

## Claims

1. An instrument for alleviating addictive drug craving, possessing a visual appearance similar to the form of an addictive drug usually consumed by an addictive drug dependent patient and comprising a pseudo addictive drug not containing the effective component of said addictive drug.

2. An instrument for alleviating addictive drug craving set forth in claim 1, wherein said addictive drug is antipsychotic agent, antianxiety agent, antimanic psychosis agent, stimulator for central nervous system, sleep inducing agent, calmative narcoleptive agent, antihistamic agent, overall cold agent, antitussive agent, anesthetic agent, lenitive agent, hallucinogenic agent, antihypnotic agent, tobacco, agent for aiding smoke prohibition, hormone, alcoholic beverage, organic solvent, or organic fuel.

3. An instrument for alleviating addictive drug craving set forth in claim 1 or claim 2, wherein said pseudo addictive drug contains physiological salt solution, grape sugar solution, distilled water, grape sugar, cane sugar, milk sugar, starch, dextrin, or saccharose.

4. An instrument for alleviating addictive drug craving set forth in any one of claims 1 - 3, wherein said pseudo addictive drug comprises a pseudo preparation possessing a visual appearance similar to the preparation of said addictive drug.

5. An instrument for alleviating addictive drug craving set forth in claim 4, wherein said pseudo preparation is aerosol, internal liquid medicine, extract, elixir, capsule, granules, pills, ophthalmic ointment, suspension, emulsion, agent for percutaneous absorption, suppository, abstract, spirit, tablet, syrup, infusion, decoction, injection, patch, tincture, ophthalmic solution, troche, ointment, poultice, aromatic water, liniment, limonade, fluidextract, lotion, catapasm, paste, collutorium, malagma, liniment, abluent, clyster, bath liquid, tea, or lincture.

6. An instrument for alleviating addictive drug craving set forth in claim 4 or claim 5, wherein said pseudo addictive drug possesses a display indicating this drug to be a counterfeit attached to the surface of said pseudo preparation.

7. An instrument for alleviating addictive drug craving set forth in any one of claims 1 - 6, wherein said pseudo addictive drug is enclosed with a pseudo container similar in visual appearance and structure to the container of said addictive drug.

8. An instrument for alleviating addictive drug craving set forth in claim 7, wherein said pseudo container is ampoule, vial, bottle, bag, syringe, sheet, stick, tube, container, pouch, or charta.

9. An instrument for alleviating addictive drug craving set forth in claim 7 or claim 8, wherein said pseudo container is made of glass, rigid plastic, flexible plastic, metal, paper, or a combination thereof.

10. An instrument for alleviating addictive drug craving set forth in any one of claims 7 - 9, wherein said pseudo addictive drug possesses a display indicating this drug to be a counterfeit attached to the visible surface of said pseudo container.

11. An instrument for alleviating addictive drug craving set forth in any one of claims 7 - 10, wherein a pseudo package similar in visible appearance and structure to the package applied to said container is applied to said pseudo package.

12. An instrument for alleviating addictive drug craving set forth in claim 11, wherein said pseudo package is a pouch, a box, or a sheet which is made of paper or plastic.

13. An instrument for alleviating addictive drug craving set forth in claim 11 or claim 12, wherein a display indicating said pseudo addictive drug to be a counterfeit is applied to the visible surface of said pseudo package.

14. A method for using an instrument for alleviating addictive drug craving which possesses a visible appearance similar in form to said addictive drug usually consumed by an addictive drug dependent patient and contains none of the active component of said addictive drug, which method comprises the first step of procedure for causing said pseudo addictive drug to be consumed by a prescribed method.

15. A method for using an instrument for alleviating addictive drug craving set forth in claim 14, which further comprises the second step of procedure for opening said pseudo container when said pseudo addictive drug is enclosed in a pseudo container similar in visible appearance and structure to the container of said addictive drug.

16. A method for using an instrument for alleviating addictive drug craving set forth in claim 15, which further comprises the third step of procedure for undoing said pseudo package when said pseudo container has applied thereto a pseudo package similar in visible appearance and structure to the package of said container.

17. A method for using an instrument for alleviating addictive drug craving set forth in claim 15 or claim 16, wherein said first step of procedure further comprises the fourth step of procedure for inserting the hollow needle of a syringe into the pseudo container opened by said second step of procedure and aspirating said pseudo addictive drug in said container with said syringe when said pseudo addictive drug is an injection.

18. A method for using an instrument for alleviating addictive drug craving set forth in claim 17, wherein said first step of procedure further comprises the fifth step of procedure for plunging into the patient's own body said hollow needle of said syringe which has aspirated said pseudo addictive drug by said fourth step of procedure and injecting said pseudo addictive drug.

19. A method for using an instrument for alleviating addictive drug craving set forth in any one of claims 14 - 18, which has part or the whole of said first - fifth steps of procedure repetitively performed for a definite period of time or steadily.

20. A method for curing dependence on an addictive drug by using an instrument for alleviation of addictive drug craving which possesses a visible appearance similar in form to said addictive drug usually consumed by an addictive drug dependent patient and contains none of the active component of said addictive drug, which comprises the first step of procedure for causing said dependent patient to consume said pseudo addictive drug by himself by a prescribed method.

21. A method for curing dependence on an addictive drug set forth in claim 20, which further comprises the second step of procedure for causing said addictive dependent patient to open said pseudo container when said addictive drug is enclosed with said pseudo container similar in visible appearance and structure to the container of said addictive drug.

22. A method for curing dependence on an addictive drug set forth in claim 21, which further comprises the third step of procedure for causing the addictive drug dependent patient to undo said pseudo package when said pseudo package has applied thereto a pseudo package similar in visible appearance and structure to the package of said container.

23. A method for curing dependence on an addictive drug set forth in claim 21 or claim 22, wherein said first step of procedure further comprises the fourth step of procedure for causing said addictive drug dependent patient to plunge the hollow needle of the syringe into the pseudo container opened by said second step of procedure and aspirate said pseudo addictive drug with said syringe when said pseudo addictive drug is an injection.

24. A method for curing dependence on an addictive drug set forth in claim 23, wherein said first step of procedure further comprises the fifth step of procedure for causing said addictive drug dependent patient to plunge into his own body said hollow needle of said syringe which has aspirated said pseudo addictive drug by said fourth step of procedure and inject said pseudo addictive drug.

25. A method for curing dependence on an addictive drug set forth in any one of claims 20 - 24, which has part or the whole of said first - fifth steps of procedure repetitively performed by said addictive drug dependent patient for a definite period of time or steadily.
